# EUROPÄISCHE PATENTSCHRIFT

(11) **EP 0 633 244 B1**
(45) Veröffentlichungstag und Bekanntmachung des Hinweises auf die Patenterteilung: **15.04.1998**
(21) Anmeldenummer: 94110458.0
(22) Anmeldetag: 05.07.1994
(51) Int. Cl.: C07C 231/02, C07C 233/18

(54) **Verfahren zur kontinuierlichen Herstellung von Polyhydroxyfettsäureamiden aus N-Alkylpolyhydroxyaminen und Fettsäurealkylestern**
Process for the continuous preparation of polyhydroxy fatty acid amide from N-alkylpolyhydroxyamines and fatty acid esters
Procédé pour la préparation en continu d'amides d'acides gras polyhydroxylées à partir d'amines polyhydroxylées N-alkylées et d'esters d'alkyl d'acides gras

(30) Priorität: 09.07.1993 DE 4322874
(43) Veröffentlichungstag der Anmeldung: 11.01.1995
(73) Patentinhaber: Clariant GmbH, 65929 Frankfurt am Main (DE)
(72) Erfinder: Aigner, Rudolf, DI, D-84508 Burgkirchen (DE); Fruth, Anton, D-84571 Reischach (DE); Keck, Helmut, D-84508 Burgkirchen (DE); Meyer, Uwe, D-84513 Töging (DE); Seitz, Hubert, Dr., D-84508 Burgkirchen (DE); Strauss, Julius, DI, D-84508 Altötting (DE); Stühler, Herbert, Dr., D-84508 Burgkirchen (DE); Vervuert, Manfred, Dr., D-87733 Markt Rettenbach (DE); Koch, Georg, DI, D-84508 Burgkirchen (DE); Vybiral, Reinhard, Dr., D-84508 Burgkirchen (DE)

(56) Entgegenhaltungen:
- WO-A-92/06073
- WO-A-94/10130
- US-A- 5 188 769

## Beschreibung

Die Erfindung betrifft ein Verfahren zur kontinuierlichen Herstellung von Polyhydroxyfettsäureamiden durch Umsetzung von N-Alkylpolyhydroxyaminen mit Fettsäurealkylestern in Gegenwart basischer Katalysatoren.

Polyhydroxyfettsäureamide sind wertvolle und vielfach einsetzbare oberflächenaktive Verbindungen. So können sie zum Beispiel als solche oder in Mischung mit anionischen, kationischen und/oder nichtionischen Tensiden als Reinigungsmittel, Waschmittel, Textilbehandlungsmittel und dergleichen eingesetzt werden, und zwar in Form von Festprodukten (zum Beispiel als Pulver, Körner oder Granulat), Lösungen, Dispersionen, Emulsionen, Pasten und dergleichen. Da Polyhydroxyfettsäureamide auch gut biologisch abbaubar sind und aus nachwachsenden Rohstoffen hergestellt werden können, haben sie in jüngster Zeit größere Bedeutung erlangt.

Bei den in Rede stehenden Polyhydroxyfettsäureamiden handelt es sich in der Regel um Verbindungen der allgemeinen Formel R-CO-NR'-Z, in der R einen Kohlenwasserstoffrest mit etwa 5 bis 30 C-Atomen, vorzugsweise 8 bis 18 C-Atomen, R' H, Alkyl oder Hydroxyalkyl mit bis zu vorzugsweise 6 C-Atomen und Z einen linearen Polyhydroxykohlenwasserstoffrest mit mindestens drei OH, die auch alkoxyliert sein können, vorzugsweise einen Zuckeralkoholrest, bedeutet. Die bevorzugten Polyhydroxyfettsäureamide entsprechen also der nachstehenden Formel 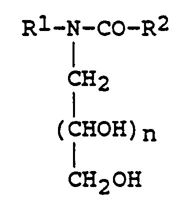 worin R¹ ein kurzkettiges Alkyl oder Hydroxyl, R² ein Fettalkyl und n vorzugsweise 3 oder 4 ist. Die Verbindungen mit n = 4, die besonders bevorzugt sind, werden als Glycamide bezeichnet und im Falle von Glucose als Hexose-Rest Glucamide.

Die Herstellung von Polyhydroxyfettsäureamiden erfolgt im allgemeinen durch Umsetzung von einem N-Alkylpolyhydroxyamin (zum Beispiel N-Alkylglucamin) mit einem Fettsäurealkylester in Gegenwart basischer Verbindungen als Katalysator. Die nachstehende Reaktionsgleichung mit N-Methylglucamin und Laurinsäuremethylester soll dies näher veranschaulichen:

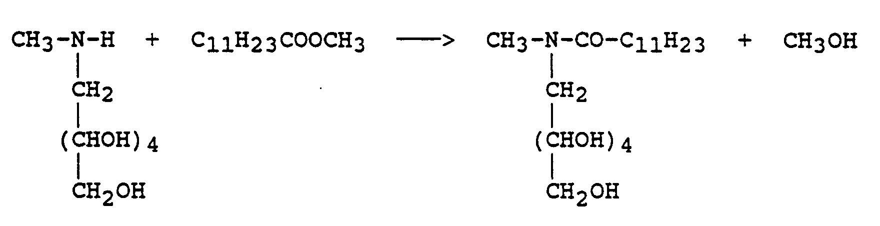

Ein derartiges Verfahren ist zum Beispiel in den Druckschriften WO 92/06071, WO 92/06072, WO 92/06073, WO 92/08687, WO 93/03004 und US 5188769 beschrieben und in WO 94/10130 vorgeschlagen.

Bei dem in WO 94/10130 vorgeschlagenen Verfahren wird eine Mischung aus der Gesamtmenge des Fettsäurealkylesters und des basischen Katalysators vorgelegt und erst dann wird die Aminkomponente zudosiert. Im Unterschied hierzu wird in dem erfindungsgemäßen Verfahren eine Mischung aus N-Alkylpolyhydroxyamin und basischem Katalysator kontinuierlich im ersten Kessel der Kaskade mit dem Fettsäurealkylester vereinigt. Erst im zweiten Kessel wird unter Vakuum und geeigneten Verweilzeiten der entstehende Alkohol abdestilliert. Das erfindungsgemäße Verfahren zeichnet sich durch hohe Umsätze (94 bis 98 %, bezogen auf N-Methylglucamin) sowie einen sehr niedrigen Gehalt an cyclischen Nebenprodukten und eine äußerst geringe Restmenge von Methanol im fertigen Produkt aus.

Nach dem bekannten Verfahren zur Herstellung von Polyhydroxyfettsäureamiden werden das N-Alkylglucamin und der Fettsäurealkylester im Molverhältnis von im wesentlichen 1 : 1 in Gegenwart eines basischen Katalysators aus der Gruppe der Alkalimetallalkoxide und Alkalimetallhydroxide bei einer Temperatur von unterhalb 135 °C umgesetzt (vergleich zum Beispiel WO 92/06073, Seite 10, Zeilen 3 bis 12). Im einzelnen wird so vorgegangen, daß das sekundäre Glucamin, der Fettsäurealkylester, die basische Katalysatorverbindung und ein Lösungsmittel miteinander gemischt und die Mischung unter Rückflußkochen bis zum gewünschten Umsatz gehalten wird, worauf das Fettsäureglucamid aus dem Reaktionsprodukt, gegebenenfalls unter Vakuumbehandlung, gewonnen wird (vergleich zum Beispiel WO 92/06073, Beispiele I bis V). Dieses Verfahren liefert einen relativ hohen Umsatz des eingesetzten N-Alkylpolyhydroxyamins und das erhaltene N-Methylglucamid ist auch relativ rein, es weist aber unter anderem die Nachteile von diskontinuierlichen Verfahrensweisen auf. In den genannten Druckschriften wird zwar darauf hingewiesen, daß das beschriebene Verfahren auch kontinuierlich durchgeführt werden kann, es wird aber nichts Weiteres gesagt, insbesondere bezüglich apparativer Ausgestaltung, Durchführung, Reaktionsbedingungen und dergleichen (vergleich zum Beispiel WO 92/06073, Seite 10, Zeilen 3 bis 12).

Die vorliegende Erfindung stellt nun ein kontinuierliches Verfahren zur Herstellung von Polyhydroxyfettsäureamiden zur Verfügung. Ausgehend von Fettsäure-C₁ bis C₄-alkylestern, vorzugsweise von Fettsäuremethylestern, soll ein hoher Umsetzungsgrad des eingesetzten N-Alkylpolyhydroxyamins erreicht werden und das erhaltene Polyhydroxyfettsäureamid soll sehr rein sein, das heißt, es soll die besonders unerwünschten Nebenprodukte, das sind cyclische Polyhydroxyverbindungen, nur noch in einer sehr geringen Menge enthalten.

Das erfindungsgemäße Verfahren zur kontinuierlichen Herstellung von Polyhydroxyfettsäureamid durch Umsetzung von N-Alkylpolyhydroxyamin und Fettsäure-C₁ bis C₄-alkylester in Gegenwart basischer Katalysatoren ist dadurch gekennzeichnet, daß die Umsetzung in zwei oder drei kaskadenförmig angeordneten Rührkesseln kontinuierlich durchgeführt und dabei so vorgegangen wird, daß (im stationären Zustand)
a₁) zunächst das N-Alkylpolyhydroxyamin mit einem Alkalimetallalkoxid oder einem Alkalimetallhydroxid oder mit einer Mischung davon in einer Menge von 0,01 bis 0,15 mol, vorzugsweise 0,05 bis 0,1 mol, pro mol N-Alkylpolyhydroxyamin umgesetzt wird zur Bereitung einer im wesentlichen wasserfreien Mischung bestehend im wesentlichen aus N-Alkylpolyhydroxyamin und Katalysator und
a₂) dem ersten Rührkessel kontinuierlich und gleichzeitig die im Schritt a₁) bereitete Mischung in Form einer fließenden Schmelze und den Fettsäure-C₁ bis C₄-alkylester in einer Menge von 1 bis 1,5 mol, vorzugsweise 1 bis 1,1 mol, pro mol des im Schritt a₁) eingesetzten N-Alkylpolyhydroxyamins zugeführt wird und im Kessel eine Temperatur von 60 bis 120 °C, vorzugsweise 65 bis 95 °C, und eine solche Verweilzeit eingehalten wird (unter Beibehaltung des anwesenden C₁ bis C₄-Alkohols), daß eine einphasige flüssige Reaktionsmischung erhalten wird,
b) die den ersten Rührkessel verlassende und in den zweiten Rührkessel eintretende einphasige flüssige Reaktionsmischung im zweiten Rührkessel bei einer Temperatur von 60 bis 100 °C, vorzugsweise 65 bis 90 °C, unter Vakuum und bei einer solchen Verweilzeit gehalten wird, daß im Falle von zwei Rührkesseln 80 bis 98 Mol-%, vorzugsweise 85 bis 95 Mol-%, vom N-Alkylpolyhydroxyamin umgesetzt sind, wobei im wesentlichen der gesamte anwesende C₁ bis C₄-Alkohol abdestilliert wird, und im Falle von drei Rührkesseln 80 bis 95 Mol-%, vorzugsweise 85 bis 93 Mol-%, vom N-Alkylpolyhydroxyamin umgesetzt sind, wobei anwesender C₁ bis C₄-Alkohol bis auf eine Restmenge von 0,1 bis 20 Gew.-%, vorzugsweise 0,5 bis 5 Gew.-%, bezogen auf Feststoffprodukt, abdestilliert wird,
c) bei dem Verfahren mit zwei Rührkesseln aus der den zweiten Rührkessel verlassenden Reaktionsmischung das Polyhydroxyfettsäureamid gewonnen wird, bei dem Verfahren mit drei Rührkesseln die den zweiten Rührkessel verlassende und in den dritten Rührkessel eintretende einphasige flüssige Reaktionsmischung im dritten Rührkessel bei einer Temperatur von 60 bis 100 °C, vorzugsweise 65 bis 90 °C, unter Vakuum und bei einer solchen Verweilzeit gehalten wird, daß 93 bis 99 Mol-%, vorzugsweise 95 bis 98 Mol-%, vom N-Alkylpolyhydroxyamin umgesetzt sind, wobei im wesentlichen der gesamte anwesende C₁ bis C₄-Alkohol abdestilliert wird, und daß
d) aus der den dritten Rührkessel verlassenden Reaktionsmischung das Polyhydroxyfettsäureamid gewonnen wird.

Beim erfindungsgemäßen Verfahren wird also in einem ersten Schritt (a₁) das N-Alkylpolyhydroxyamin mit Alkalimetallalkoxid oder Alkalimetallhydroxid oder mit einer Mischung davon in einer Menge von 0,01 bis 0,15 mol, vorzugsweise 0,05 bis 0,1 mol, pro mol N-Alkylpolyhydroxyamin umsetzt zur Bereitung einer im wesentlichen wasserfreien Mischung bestehend im wesentlichen aus N-Alkylpolyhydroxyamin und dem gebildeten und als Katalysator fungierenden Alkalimetallsalz von N-Alkylpolyhydroxyamin. Diese Mischung und Fettsäurealkylester in einer Menge von 1 bis 1,5 mol, vorzugsweise 1 bis 1,1 mol, pro mol des zur Bereitung der Mischung eingesetzten N-Alkylpolyhydroxyamins werden dann kontinuierlich und gleichzeitig dem ersten Rührkessel zugeführt. Die Umsetzung im ersten, zweiten und dritten Kessel wird dann wie oben beschrieben fortgesetzt.

Zum Vakuum, bei dem der zweite und dritte Rührkessel betrieben wird, sei noch folgendes gesagt: Im Falle von zwei Rührkesseln wird im zweiten Kessel im allgemeinen ein Vakuum von 5 bis 150 mbar, vorzugsweise 10 bis 50 mbar, gehalten und im Falle von drei Rührkesseln ein solches von 50 bis 600 mbar im zweiten, vorzugsweise von 100 bis 500 mbar, und ein solches von 5 bis 100 mbar, vorzugsweise 10 bis 50 mbar, im dritten Kessel.

Nachdem es das Ziel des erfindungsgemäßen Verfahrens ist, auch schon im ersten Rührkessel eine möglichst niedrige Temperatur zu halten und ständig eine einphasige flüssige Reaktionsmischung (eine homogene Phase oder klare Lösung) vorliegen zu haben (das heißt, daß noch nicht umgesetztes N-Alkylpolyhydroxyamin vollständig gelöst vorliegt), kann es unter Umständen erforderlich sein, zusätzlich zu dem durch die Amidierungsreaktion freigesetzten Alkohol weiteren Alkohol (Methanol) als Lösungsmittel einzubringen. Diese Menge an zusätzlichem Alkohol, den man dem ersten und häufig auch noch dem zweiten Rührkessel zuführt, liegt insgesamt im allgemeinen bei 0,5 bis 20 mol, vorzugsweise bei 1 bis 10 mol, pro mol des dem ersten Kessel zugeführten N-Alkylpolyhydroxyamins.

Die beschriebene Mischung aus N-Alkylpolyhydroxyamin und einem Alkalimetallsalz davon in katalytischer Menge wird vorzugsweise in Form einer fließenden Schmelze bei circa 125 bis 135 °C dem ersten Rührkessel zugeführt. Die Bereitung der Mischung im einzelnen wird bevorzugt in der Weise durchgeführt, daß man das N-Alkylpolyhydroxyamin und die Alkalimetallverbindung jeweils in Form einer 10 bis 60gew.%igen, vorzugsweise 15 bis 50gew.%igen, wäßrigen Lösung einsetzt (das N-Alkylpolyhydroxyamin fällt aufgrund seiner Herstellungsart im allgemeinen in Form der genannten Lösungen an), die beiden Lösungen zusammenmischt, vorzugsweise bei Raumtemperatur, und die wäßrige Mischung (Gesamtlösung) durch Entfernen des Wassers trocknet. Das Lösungsmittel Wasser wird bevorzugt soweit entfernt, daß nur noch höchstens 0,5 Gew.-% Wasser vorliegen, vorzugsweise höchstens 0,2 bis 0,3 Gew.-%, bezogen auf die trockene (entwässerte) Mischung. Die Entwässerung wird vorzugsweise dadurch erreicht, daß aus der genannten wäßrigen Gesamtlösung bei circa 90 bis 135 °C das Wasser unter Vakuum abdestilliert wird. Die Entwässerung kann mit Hilfe üblicher Dünnschichtverdampfer, Fallfilmverdampfer oder Strippapparaturen durchgeführt werden. Es wird davon ausgegangen, daß die nach der Wasserentfernung erhaltene Mischung im wesentlichen aus dem eingesetzten N-Alkylpolyhydroxyamin als Hauptbestandteil und gebildeten Alkalimetallsalz von N-Alkylpolyhydroxyamin besteht, wobei auch noch weitere basische (alkalische) und ebenfalls als Katalysator wirkende Alkalimetallverbindungen wie nicht umgesetzte Alkalimetallausgangsverbindung und/oder Modifikationen davon, zum Beispiel Alkalimetalloxid, vorliegen können. Dieses Produkt (Mischung) ist bei Raumtemperatur fest, bei 115 bis 135 °C eine mehr oder weniger flüssige Schmelze und enthält im allgemeinen nur noch die obengenannte Restwassermenge von höchstens 0,5 Gew.-%, vorzugsweise höchstens 0,2 bis 0,3 Gew.-%, bezogen auf das Gewicht des Produktes.

Es hat sich als vorteilhaft erwiesen, wenn man im ersten oder zweiten Rührkessel oder in beiden, vorzugsweise nur im zweiten, ein viskositätssenkendes Mittel einbringt. Solche Mittel sind vorzugsweise Ethylenglykol, Propylenglykol oder C₈ bis C₁₈-Fettalkohole oder Oxethylate davon mit 1 bis 5 Ethylenoxid-Einheiten, wobei Ethylenglykol, Propylenglykol oder eine Mischung davon bevorzugt ist. Die Menge kann in weiten Grenzen variieren. Sie beträgt im allgemeinen 1 bis 50 Gew.-%, vorzugsweise 2 bis 30 Gew.-%, bezogen auf Feststoffprodukt.

Die Einleitung der erfindungsgemäßen kontinuierlichen Umsetzung, das heißt die Bereitung einer einphasigen flüssigen Startmischung im ersten Rührkessel, kann auf verschiedene Art und Weise erfolgen. So kann man den Fettsäureester vorlegen, auf Reaktionstemperatur erhitzen und in den erhitzten Ester die beschriebene Schmelze aus Alkalimetallverbindung und N-Alkylpolyhydroxyamin und gegebenenfalls Methanol einbringen, worauf die Mischung so lange bei Reaktionstemperatur gehalten wird (ohne Entfernung des Lösungsmittels), bis man die angestrebte einphasige flüssige Reaktionsmischung erhält. Die genannten Komponenten können auch in einer anderen Reihenfolge in den ersten Kessel gebracht werden. Man kann gegebenenfalls auch alle genannten Komponenten im ersten Rührkessel vorlegen und auf Reaktionstemperatur halten, bis die anfangs disperse Reaktionsmischung das angestrebte Aussehen hat.

Die erfindungsgemäße Umsetzung wird bei Atmosphärendruck oder dem im Rührkessel sich einstellenden Druck durchgeführt, abgesehen von dem zur schnelleren Austragung des Alkohols aus dem Reaktionsgemisch beschriebenen Vakuum. Wie bereits erwähnt, wird in jedem nachfolgenden Rührkessel auf eine im Vergleich zum vorangehenden Kessel höhere Polyhydroxyfettsäureamid-Menge umgesetzt. Es ist auch bevorzugt, in jedem nachfolgenden Rührkessel bei einer im Vergleich zum vorangehenden Kessel höchstens gleichen (also nicht höheren) und vorzugsweise etwas niedrigeren Temperatur (zweckmäßigerweise 1 bis 5 °C) umzusetzen. Das im letzten Rührkessel homogene flüssige und bei Raumtemperatur feste Produkt besteht im wesentlichen aus Polyhydroxyfettsäureamid, nicht umgesetztem N-Alkylpolyhydroxyamin und der gegebenenfalls eingesetzten viskositätssenkenden Flüssigkeit. In der Regel wird man das erhaltene Produkt in zusätzlicher Flüssigkeit (Lösungsmittel) und/oder Wasser aufnehmen, womit nicht nur eine rasche Abkühlung, sondern auch ein bei Raumtemperatur gut fließendes und damit gut handhabbares Produkt erhalten wird. Das Polyhydroxyfettsäureamid kann auch in die Form von Schuppen, Körnern, Granulat oder Pulver gebracht werden.

Das erfindungsgemäße Verfahren weist eine Reihe von Vorteilen auf. Das lineare Polyhydroxyfettsäureamid wird in nahezu quantitativer Ausbeute erhalten. Es enthält nur sehr wenig störende Nebenprodukte wie cyclische Polyhydroxyverbindungen. Mit dem erfindungsgemäßen Verfahren ist es also möglich, Polyhydroxyfettsäureamid in hoher Ausbeute (Raum-Zeit-Ausbeute) und in hoher Reinheit herzustellen. Das lineare Fettsäureamid (Schmelze) ist leicht gelb gefärbt, hat also auch die erwünschte gute Farbe. Das erfindungsgemäße Verfahren ist kontinuierlich und kann in apparativ einfachen Rührkesseln durchgeführt werden. Daraus resultiert ein weiterer wesentlicher Vorteil, nämlich der, daß die Umsetzung im Hinblick auf Automatisierung, Steuerung und Einhaltung konstanter Reaktionsbedingungen, was für eine gleichmäßige Produktqualität klarerweise von großer Bedeutung ist, optimal durchgeführt werden kann. Ein anderer wesentlicher Vorteil liegt darin, daß in der stationären Phase der Umsetzung oft schon der bei der Umsetzung entstehende Alkohol als Lösungsmittel ausreicht und stets vollständige Homogenität vorliegt. Dies gewährleistet eine hohe Reaktionsgeschwindigkeit und gleichzeitig den hohen Umsetzungsgrad und die hohe Reinheit des Produktes. Das klare homogene Reaktionsgemisch erlaubt von Anfang an relativ niedrige Reaktionstemperaturen, was sich wiederum günstig für die Reinheit des Polyhydroxyfettsäureamids auswirkt.

Im folgenden sei zu den beim erfindungsgemäßen Verfahren einzusetzenden basischen Verbindungen sowie zum N-Alkylpolyhydroxyamin und zum Fettsäurealkylester noch folgendes gesagt: Die basischen Verbindungen sind vorzugsweise Alkalimetallverbindungen in Form von Alkalimetallalkoxiden und/oder Alkalimetallhydroxiden. Die Alkalimetallalkoxide sind vorzugsweise C₁ bis C₄-Alkalimetallalkoxide, wobei die C₁ bis C₃-Alkoxide bevorzugt sind. Besonders bevorzugt ist das Alkalimetallmethylat. Das Alkalimetall ist vorzugsweise Natrium oder Kalium. Die Alkalimetallhydroxide sind vorzugsweise Natriumhydroxid und Kaliumhydroxid.

Die Ausgangsverbindungen N-Alkylpolyhydroxyamin und Fettsäurealkylester sind ebenfalls bekannte Verbindungen und im Handel erhältlich. Sie sind ferner in der eingangs genannten Druckschrift WO 92/06073 ausführlich beschrieben, die hier miteinbezogen wird. Die N-Alkylpolyhydroxyamine können Alkylreste und Polyhydroxyreste verschiedenster Art enthalten. Was den Polyhydroxyanteil betrifft, stammt dieser vorzugsweise von Polyhydroxyverbindungen aus der Gruppe der reduzierenden Zucker oder reduzierenden Zuckerderivate. Bevorzugte reduzierende Zuckerverbindungen sind die Monosaccharide, vorzugsweise Pentosen und Hexosen, und die Oligosaccharide, vorzugsweise Disaccharide und Trisaccharide. Beispiele für Monosaccharide sind Fructose, Glucose, Galaktose, Mannose, Sorbose und Talose als Hexosen und Arabinose, Ribose und Xylose als Pentosen. Von den Monosacchariden sind die Hexosen bevorzugt. Beispiele für Oligosaccharide (Polysaccharide) sind Lactose, Maltose, Maltotriose und dergleichen. Von den Oligosacchariden sind die Disaccharide bevorzugt. Besonders bevorzugte Polyhydroxyverbindungen sind die (reduzierenden) Hexosen, insbesondere die Glucose. Der Alkylrest im N-Monoalkylpolyhydroxyamin kann auch ein Hydroxyalkylrest, zum Beispiel -CH₂CH₂OH, sein. Er ist vorzugsweise ein C₁ bis C₄-Alkyl, zum Beispiel Methyl, Ethyl, Propyl oder Isopropyl. Bevorzugte N-Alkylpolyhydroxyamine sind also die N-C₁ bis C₃-Glycamine, vorzugsweise von Fructose, Glucose, Galaktose, Mannose, Sorbose oder Talose oder von deren Gemischen. Besonders bevorzugte N-Alkylpolyhydroxyamine sind die N-C₁ bis C₃-Glucamine, wobei das N-Methylglucamin ganz besonders bevorzugt ist.

Die Fettsäurealkylester sind vorzugsweise Fettsäure-C₁ bis C₄-alkylester, wobei Methyl, Ethyl, Propyl oder Isopropyl bevorzugt sind. Die Fettsäuremethylester sind besonders bevorzugt. Der Fettsäurerest (die Acylgruppe) hat im allgemeinen 6 bis 24 C-Atome, vorzugsweise 8 bis 18 C-Atome. Er kann gesättigt oder ungesättigt (vorzugsweise ein- bis dreifach ungesättigt) sein. Als Beispiele seien die Acylreste von Capryl-, Caprin-, Laurin-, Palmitin-, Stearin- und Ölsäure genannt sowie Cocosacyl, Talgacyl, vorzugsweise gehärtetes Talgacyl, und dergleichen. Der Fettsäurerest stellt häufig eine Mischung von zwei oder mehreren Acylgruppen dar, zum Beispiel C₁₂ und C₁₄-Acyl (C_{12/14}), C₁₆ und C₁₈-Acyl (C_{16/18}) oder C₁₂ bis C₁₈-Acyl.

Die Erfindung wird nun an Beispielen noch näher erläutert.

Die nachstehend benützte Abkürzung "MG" bedeutet Molekulargewicht.

### Beispiel 1

### Produktzufuhr zum ersten Kessel:

- 1,00 mol: N-Methylglucamin enthaltend 0,07 mol Katalysator
- 1,06 mol: C_{12/14}-Fettsäuremethylester (MG = 220)
- 2,00 mol: Methanol

### Produktzufuhr zum zweiten Kessel:

- 0,50 mol: Propylenglykol (10 Gew.-%)

Die Mischung von N-Methylglucamin und 0,07 mol Katalysator wurde in der Weise bereitet, daß 1 mol N-Methylglucamin und 0,07 mol NaOH jeweils in Form einer 35gew.%igen wäßrigen Lösung vermischt wurden und die Gesamtlösung mit Hilfe eines Dünnschichtverdampfers bei bis zu 130 °C und einem Vakuum von 50 mbar bis auf 0,3 Gew.-% Restwasser entwässert wurde. Das Produkt wird als Schmelze (circa 130 °C) eingesetzt.

### Durchführung:

Um die kontinuierliche Herstellung von N-Methylglucamid zu starten, werden zunächst im ersten Rührkessel 3 mol C_{12/14}-Fettsäuremethylester vorgelegt und auf etwa 95 °C erhitzt. In den erhitzten Ester werden 3 mol von der oben beschriebenen Schmelze, bezogen auf N-Methylglucamin, eingebracht. Die Mischung wird auf etwa 87 °C und dem sich einstellenden Druck gehalten, bis eine einphasige flüssige (klare homogene) Reaktionsmischung erhalten wird. Sobald im ersten Rührkessel diese Mischung vorliegt, beginnt die kontinuierliche Arbeitsweise, das heißt dem ersten Kessel werden kontinuierlich und gleichzeitig pro Stunde die oben unter "Produktzufuhr zum ersten Kessel" angegebenen Produkte unter Aufrechterhaltung der genannten Temperatur von etwa 87 °C zugeführt. Die den ersten Kessel verlassende und in den zweiten Kessel eintretende klare homogene Reaktionsmischung (die beiden Kessel der Kaskade sind gefüllt und im stationären Zustand) weist einen Umsatz von 77,4 Mol-% auf, bezogen auf N-Methylglucamin. Die mittlere Verweilzeit im ersten Kessel beträgt 1,6 Stunden. Im zweiten Rührkessel wird die Reaktionsmischung bei 80 °C und einem Vakuum von 23 mbar gehalten, wobei das Methanol abdestilliert wird. Dem zweiten Kessel wird kontinuierlich pro Stunde die oben angegebene Propylenglykolmenge zur Senkung der Viskosität der Reaktionsmischung im zweiten Kessel zudosiert. Die mittlere Verweilzeit im zweiten Kessel, in dem die Amidierungsreaktion weitergeht, beträgt 2 Stunden. Das den zweiten Kessel verlassende klare Produkt weist einen Umsatz von 93,9 Mol-% auf, bezogen auf N-Methylglucamin.

### Beispiel 2

### Produktzufuhr zum ersten Kessel:

- 1,0 mol: N-Methylglucamin enthaltend 0,15 mol Katalysator
- 1,3 mol: C_{12/14}-Fettsäuremethylester (MG = 220)
- 20,0 mol: Methanol

### Produktzufuhr zum zweiten Kessel:

- 0,5 mol: Propylenglykol (10 Gew.-%)

Die Mischung von N-Methylglucamin und 0,15 mol Katalysator wurde in der Weise bereitet, daß 1 mol N-Methylglucamin und 0,15 mol NaOH jeweils in fester Form vermischt und die Mischung aufgeschmolzen wurde.

### Durchführung:

Um die kontinuierliche Herstellung von N-Methylglucamid zu starten, wird zunächst wie in Beispiel 1 vorgegangen, wobei eine Temperatur von 60 °C eingestellt und gehalten wird. Sobald im ersten Rührkessel die beschriebene Reaktionsmischung vorliegt, beginnt die kontinuierliche Arbeitsweise, das heißt dem ersten Kessel werden kontinuierlich und gleichzeitig pro Stunde die oben unter "Produktzufuhr zum ersten Kessel" angegebenen Produkte unter Aufrechterhaltung der genannten Temperatur von etwa 60 °C zugeführt. Die den ersten Kessel verlassende und in den zweiten Kessel eintretende klare homogene Reaktionsmischung (die drei Kessel der Kaskade sind gefüllt und im stationären Zustand) weist einen Umsatz von 65,3 Mol-% auf, bezogen auf N-Methylglucamin. Die mittlere Verweilzeit im ersten Kessel beträgt 1,3 Stunden. Im zweiten Rührkessel wird die Reaktionsmischung bei 60 °C und einem Vakuum von 600 mbar gehalten, wobei das Methanol abdestilliert wird. Dem zweiten Kessel wird kontinuierlich pro Stunde die oben angegebene Propylenglykolmenge zur Senkung der Viskosität der Reaktionsmischung im zweiten Kessel zudosiert. Die mittlere Verweilzeit im zweiten Kessel, in dem die Amidierungsreaktion weitergeht, beträgt 2,6 Stunden. Die den zweiten Kessel verlassende und in den dritten Kessel eintretende einphasige flüssige Reaktionsmischung weist einen Umsatz von 85,4 Mol-% auf, bezogen auf N-Methylglucamin. Im dritten Rührkessel wird die Reaktionsmischung bei 60 °C und einem Vakuum von 94 mbar gehalten, wobei Methanol weiter abdestilliert wird. Die mittlere Verweilzeit im dritten Rührkessel, in dem die Amidierungsreaktion noch weitergeführt wird, beträgt 3,3 Stunden. Das den dritten Kessel verlassende klare Produkt weist einen Umsatz von 97,5 Mol-% auf, bezogen auf N-Methylglucamin.

Zu den beiden Beispielen sei noch gesagt, daß der erreichte Umsetzungsgrad vom eingesetzten N-Alkylpolyhydroxyamin sehr hoch und das erhaltene lineare Polyhydroxyfettsäureamid sehr rein ist. Der Gehalt an cyclischen Verbindungen liegt im Bereich von kleiner 200 ppm bis höchstens 1000 ppm (die Bestimmung erfolgte durch quantitative Dünnschichtchromatographie). Das Polyhydroxyfettsäureamid enthält auch nur sehr wenig Rest-Methanol, da der Alkohol im zweiten Kessel (im Falle von zwei Rührkesseln) oder im dritten Kessel (im Falle von drei Rührkesseln) möglichst vollständig destillativ entfernt wird.

## Patentansprüche

1. Verfahren zur kontinuierlichen Herstellung von Polyhydroxyfettsäureamid durch Umsetzung von N-Alkylpolyhydroxyamin und Fettsäure-C₁ bis C₄-alkylester in Gegenwart basischer Katalysatoren, dadurch gekennzeichnet, daß die Umsetzung in zwei oder drei kaskadenförmig angeordneten Rührkesseln kontinuierlich durchgeführt und dabei so vorgegangen wird, daß
a₁) zunächst das N-Alkylpolyhydroxyamin mit einem Alkalimetallalkoxid oder einem Alkalimetallhydroxid oder mit einer Mischung davon in einer Menge von 0,01 bis 0,15 mol pro mol N-Alkylpolyhydroxyamin umgesetzt wird zur Bereitung einer im wesentlichen wasserfreien Mischung bestehend im wesentlichen aus N-Alkylpolyhydroxyamin und Katalysator und
a₂) dem ersten Rührkessel kontinuierlich und gleichzeitig die im Schritt a₁) bereitete Mischung in Form einer fließenden Schmelze und den Fettsäure-C₁ bis C₄-alkylester in einer Menge von 1 bis 1,5 mol pro mol des im Schritt a₁) eingesetzten N-Alkylpolyhydroxyamins zugeführt wird und im Kessel eine Temperatur von 60 bis 120 °C und eine solche Verweilzeit eingehalten wird, daß eine einphasige flüssige Reaktionsmischung erhalten wird,
b) die den ersten Rührkessel verlassende und in den zweiten Rührkessel eintretende einphasige flüssige Reaktionsmischung im zweiten Rührkessel bei einer Temperatur von 60 bis 100 °C, unter Vakuum und bei einer solchen Verweilzeit gehalten wird, daß im Falle von zwei Rührkesseln 80 bis 98 Mol-% vom N-Alkylpolyhydroxyamin umgesetzt sind, wobei im wesentlichen der gesamte anwesende C₁ bis C₄-Alkohol abdestilliert wird, und im Falle von drei Rührkesseln 80 bis 95 Mol-% vom N-Alkylpolyhydroxyamin umgesetzt sind, wobei anwesender C₁ bis C₄-Alkohol bis auf eine Restmenge von 0,1 bis 20 Gew.-%, bezogen auf Feststoffprodukt, abdestilliert wird,
c) bei dem Verfahren mit zwei Rührkesseln aus der den zweiten Rührkessel verlassenden Reaktionsmischung das Polyhydroxyfettsäureamid gewonnen wird, bei dem Verfahren mit drei Rührkesseln die den zweiten Rührkessel verlassende und in den dritten Rührkessel eintretende einphasige flüssige Reaktionsmischung im dritten Rührkessel bei einer Temperatur von 60 bis 100 °C, unter Vakuum und bei einer solchen Verweilzeit gehalten wird, daß 93 bis 99 Mol-% vom N-Alkylpolyhydroxyamin umgesetzt sind, wobei im wesentlichen der gesamte anwesende C₁ bis C₄-Alkohol abdestilliert wird, und daß
d) aus der den dritten Rührkessel verlassenden Reaktionsmischung das Polyhydroxyfettsäureamid gewonnen wird.

2. Verfahren nach Anspruch 1, dadurch gekennzeichnet, daß dem ersten Rührkessel Methanol zugeführt wird in einer Menge von 0,5 bis 20 mol pro mol des dem ersten Rührkessel zugeführten N-Alkylpolyhydroxyamins.

3. Verfahren nach einem oder mehreren der Ansprüche 1 bis 2, dadurch gekennzeichnet, daß dem zweiten Rührkessel ein viskositätssenkendes Mittel zugeführt wird, ausgewählt aus der Gruppe bestehend aus Ethylenglykol, Propylenglykol oder C₈ bis C₁₈-Fettalkoholen oder Oxethylaten davon mit 1 bis 5 Ethylenoxid-Einheiten in einer Menge von 1 bis 50 Gew.-%, bezogen auf Feststoffprodukt.

4. Verfahren nach einem oder mehreren der Ansprüche 1 bis 2, dadurch gekennzeichnet, daß dem zweiten Rührkessel Ethylenglykol, Propylenglykol oder eine Mischung davon als viskositätssenkendes Mittel zugeführt wird in einer Menge von 1 bis 50 Gew.-%, bezogen auf Feststoffprodukt.

5. Verfahren nach einem oder mehreren der Ansprüche 1 bis 4, dadurch gekennzeichnet, daß als N-Alkylpolyhydroxyamin N-Methylglucamin, als Fettsäurealkylester Fettsäuremethylester, als Alkalimetallalkoxid Kalium- oder Natriummethylat und als Alkalimetallhydroxid Kalium- oder Natriumhydroxid eingesetzt wird.

## Claims

1. A process for the continuous preparation of polyhydroxy-fatty amide by reaction of N-alkylpolyhydroxyamine and C₁ to C₄ alkyl ester of fatty acid in the presence of basic catalysts, which comprises continuously carrying out the reaction in two or three stirred tanks arranged in a cascade and carrying it out in this case in such a way that
a₁) the N-alkylpolyhydroxyamine is first reacted with an alkali metal alkoxide or an alkali metal hydroxide or with a mixture thereof in an amount of 0.01 to 0.15 mol per mole of N-alkylpolyhydroxyamine to prepare an essentially anhydrous mixture essentially comprising N-alkylpolyhydroxyamine and catalyst and
a₂) the mixture prepared in step a₁) in the form of a flowing melt and the C₁ to C₄ alkyl ester of fatty acid in an amount of 1 to 1.5 mol per mole of N-alkylpolyhydroxyamine used in step a₁) are continuously and simultaneously fed to the first stirred tank and in the tank a temperature of 60 to 120°C is maintained and a residence time is maintained so that a single-phase liquid reaction mixture is obtained,
b) the single-phase liquid reaction mixture leaving the first stirred tank and entering the second stirred tank is kept in the second stirred tank at a temperature of 60 to 100°C, under vacuum and for a residence time such that, in the case of two stirred tanks, 80 to 98 mol% of the N-alkylpolyhydroxyamine are reacted, essentially all of the C₁ to C₄ alcohol present being distilled off, and, in the case of three stirred tanks, 80 to 95 mol% of the N-alkylpolyhydroxyamine are reacted, C₁ to C₄ alcohol present being distilled off down to a residual amount of 0.1 to 20% by weight, based on solid product,
c) in the process having two stirred tanks, the polyhydroxy-fatty amide is isolated from the reaction mixture leaving the second stirred tank; in the process having three stirred tanks, the single-phase liquid reaction mixture leaving the second stirred tank and entering the third stirred tank is kept in the third stirred tank at a temperature of 60 to 100°C, under vacuum and for a residence time such that 93 to 99 mol% of the N-alkylpolyhydroxyamine are reacted, essentially all of the C₁ to C₄ alcohol present being distilled off, and that
d) the polyhydroxy-fatty amide is isolated from the reaction mixture leaving the third stirred tank.

2. The process as claimed in claim 1, wherein methanol is fed to the first stirred tank in an amount of 0.5 to 20 mol per mole of the N-alkylpolyhydroxyamine fed to the first stirred tank.

3. The process as claimed in one or more of claims 1 to 2, wherein a viscosity-decreasing agent selected from the group comprising ethylene glycol, propylene glycol or C₈ to C₁₈ fatty alcohols or ethoxylates thereof having 1 to 5 ethylene oxide units is fed to the second stirred tank in an amount of 1 to 50% by weight, based on solid product.

4. The process as claimed in one or more of claims 1 to 2, wherein ethylene glycol, propylene glycol or a mixture thereof as a viscosity-decreasing agent is fed to the second stirred tank in an amount of 1 to 50% by weight, based on solid product.

5. The process as claimed in one or more of claims 1 to 4, wherein the N-alkylpolyhydroxyamine used is N-methylglucamine, the alkyl ester of fatty acid used is a methyl ester of fatty acid, the alkali metal alkoxide used is potassium methylate or sodium methylate and the alkali metal hydroxide used is potassium hydroxide or sodium hydroxide.

## Revendications

1. Procédé pour la préparation en continu d'un amide d'acide gras polyhydroxylique par réaction de la N-alkylpolyhydroxyamine et d'un acide gras en C₁ à C₄ en présence de catalyseurs basiques, caractérisé en ce que l'on met en oeuvre la réaction en continu dans deux ou trois agitateurs disposés en cascade et pour ce faire, on opère en ce que
a₁) on fait réagir d'abord la N-alkylpolyhydroxylamine avec un oxyde de métal alcalin ou un hydroxyde de métal alcalin ou un mélange de ces substances, dans une quantité de 0,01 à 0,15 mole de N-alkylpolyhydroxylamine pour la préparation d'un mélange essentiellement anhydre constitué essentiellement de la N-alkylpolyhydroxylamine et du catalyseur et
a₂) on introduit dans le premier agitateur, en continu et simultanément, le mélange préparé à l'étape a₁) sous forme d'une matière fondue fluide et l'ester d'alkyle d'acide gras en C₁ à C₄ dans une quantité de 1 à 1,5 mole par mole de N-alkylpolyhydroxyalamine et on maintient dans le récipient une température de 60 à 120 °C et une durée de séjour telle qu'on obtient un mélange réactionnel monophasique liquide,
b) le mélange réactionnel monophasique liquide quittant le premier agitateur et entrant dans le deuxième agitateur est maintenu dans le deuxième agitateur à une température de 60 à 100 °C, sous vide et pendant une durée de séjour telle que, dans le cas de deux agitateurs, 80 à 98 % en mole de la N-alkylpolyhydroxylamine réagissent, en chassant essentiellement la totalité d'alcool en C₁ à C₄ présent, et dans le cas de trois agitateurs, 80 à 95 % en mole de N-alkyl-polyhydroxylamine réagissent en chassant par distillation l'alcool en C₁ à C₄ présent, jusqu'à une teneur résiduelle de 0,1 à 20 % en poids, par rapport au produit solide,
c) dans le procédé avec deux agitateurs, on obtient l'amide d'acide gras polyhydroxylique à partir du mélange réactionnel quittant le deuxième agitateur, dans le procédé avec trois agitateurs, on fait réagir le mélange réactionnel monophasique liquide quittant le deuxième agitateur et entrant dans le troisième agitateur, à une température de 60 à 100 °C, sous vide et en le maintenant pendant une durée de séjour telle que 93 à 99 % en mole de N-alkylpolyhydrolyamine aient réagi, en chassant essentiellement par distillation la totalité d'alcool en C₁ à C₄ présent et en ce que
d) l'on obtient l'amide d'acide gras polyhydroxylique à partir du mélange réactionnel quittant le troisième agitateur.

2. Procédé selon la revendication 1, caractérisé en ce que l'on alimente en méthanol dans le premier agitateur, dans une quantité de 0,5 à 20 moles par mole de N-alkylpolyhydroxylamine alimentée au premier agitateur.

3. Procédé selon une ou plusieurs des revendications 1 ou 2, caractérisé en ce que l'on alimente le deuxième agitateur en un agent réducteur de viscosité pris parmi l'éthylène glycol, le propylène glycol ou des alcools gras en C₈ à C₁₈ ou de leurs éthoxylates avec 1 à 5 motifs d'oxyde d'éthylène, dans une quantité de 1 à 50 % en poids, par rapport au produit solide.

4. Procédé selon une ou plusieurs des revendications 1 ou 2, caractérisé en ce que l'on alimente le deuxième agitateur en éthylène glycol, en propylène glycol ou en un mélange de ces composés en tant qu'agent réducteur de viscosité, dans une quantité de 1 à 50 % en poids, par rapport au produit solide.

5. Procédé selon une ou plusieurs des revendications 1 à 4, caractérisé en ce que l'on utilise la N-méthylglucamine en tant que N-alkylpolyhydroxylamine, un ester méthylique d'acide gras en tant qu'ester d'alkyle d'acide gras, le méthylate de potassium ou de sodium en tant qu'oxyde de métal alcalin et l'hydroxyde de potassium ou de sodium en tant qu'hydroxyde de métal alcalin.
